# EUROPEAN PATENT APPLICATION

(11) **EP 2 508 193 A1**
(43) Date of publication of application: **10.10.2012**
(21) Application number: 11161239.6
(22) Date of filing: 05.04.2011
(51) Int. Cl.: A61K 36/899, A61K 38/11, A61P 37/08, A61K 36/88, A61K 36/8998

(54) **Use of a hypoallergenic cereal composition for inducing specific oral tolerance**

(71) Applicant: Nestec S.A., 1800 Vevey (CH)
(72) Inventor: Mercenier, Annick, 1030 Bussigny (CH); Ortega, Geraldine, 1350 Orbe (CH)
(74) Representative: Cogniat, Eric Jean Marie

(57) **Abstract**

A partial hydrolysate of cereal protein for use in inducing specific oral tolerance in a young mammal with allergy to said cereal protein, wherein the hydrolysate has a degree of hydrolysis between 9 and 18%, is disclosed.

## Description

### Field of the Invention

This invention relates to the use of a hypoallergenic cereal composition for inducing specific oral tolerance to cereal, especially wheat or rice, in a young mammal with IgE-mediated allergy to cereal.

### Background to the Invention

Food allergies, of which the first to occur in life is cows' milk allergy, are caused, in most cases, by a reaction to the proteins in the food. In the early years of life the immune system is still developing and may fail to develop tolerance to dietary antigens (this may also be described as insufficient induction of oral tolerance). The result is that the baby or child or young animal mounts an exaggerated immune response to the dietary protein and develops an allergic response to it. Food allergies may affect not only humans but also other mammals such as dogs and cats.

Usually, food hypersensitivity appears just after a susceptible or an at-risk baby, child or young animal first encounters a new food containing potential allergens. Apart from its mother's milk, the first dietary proteins generally encountered by human babies at least are cows' milk proteins and, as noted above, cows' milk allergy is the most common food allergy in human babies. It is generally accepted that babies with established cows' milk allergy have an increased risk of developing atopic diseases and allergies to other dietary proteins such as egg and cereal proteins but even those babies who have successfully developed oral tolerance to cows' milk proteins may subsequently develop allergies to other dietary proteins such as egg and cereal proteins when these are introduced into the diet at weaning. These allergies may manifest themselves clinically as atopic diseases such as atopic dermatitis, eczema and asthma.

From a dietary point of view there are two ways to treat an established allergy - either foods containing the allergen must be avoided altogether, or the foods must be treated to decrease their allergenic potential, for example by extensive hydrolysis. Infant formulas containing extensively hydrolysed cows' milk proteins (peptides consisting of not more than five amino acids) are manufactured for this latter purpose. Similarly it has already been proposed, in US Patent No. 6403142 for example, to prepare petfoods with reduced allergenicity for companion animals where it is suspected that the animal has developed a food allergy.

As noted above, apart from their mothers' milk, the first dietary proteins generally encountered by young mammals are milk proteins from other animals such as cows' milk in the case of human infants. Generally, the next dietary protein encountered is cereal protein which is introduced at the start of weaning, typically in the form of infant cereals for human infants. Cereal proteins may also provoke allergic reactions when first introduced into the diet of a young mammal even if milk proteins have already been successfully introduced. In particular the proteins albumin, globulin, glutenin and gliadin in wheat have been associated with the development of allergy in human infants. The specific allergenicity profile of the cereal proteins coupled with the particular characteristics of these proteins induce a specific need for developing specific processes and products addressing their allergenicity.

Some attention has been shed on the primary prevention of establishment of allergic reactions to cereal proteins, for instance as disclosed in WO 2009/077457, ie to reduce the risk of developing the allergy in the first place.

As noted above, once an allergic condition is installed, foods containing the allergen must be avoided altogether, or the foods must be treated to decrease their allergenic potential, for example by extensive hydrolysis. In some circumstances, it may also be possible to mitigate or reduce the allergic reaction by inducing specific oral tolerance to the allergen.

Hence, it is an object of the present invention to provide a hypoallergenic cereal preparation, in the form of a liquid or dry product easily reconstituted in water or milk, for use in desensitisation against a declared food allergy or induction of oral tolerance to said allergenic protein, such as cereal protein.

### Summary of the Invention

Accordingly, in a first aspect, the present invention provides a partial hydrolysate of cereal protein for use in inducing specific oral tolerance in a young mammal with allergy to said cereal protein, wherein the hydrolysate has a degree of hydrolysis between 9 and 18%.

In a second aspect, the invention provides a cereal flour for use in inducing specific oral tolerance in a young mammal with allergy to said cereal protein, in which the protein is partially hydrolysed and has a degree of hydrolysis between 9 and 18%.

In a third aspect, the invention provides a cereal product for use in inducing specific oral tolerance in a young mammal with allergy to said cereal protein, said cereal product comprising, by percentage weight of dry matter, 1 to 100% cereal flour as defined above, preferably 15 to 100% flour in which the protein is partially hydrolysed and has a degree of hydrolysis between 9 and 18%, 0 to 40% sugar(s), 0 to 30% starch, and 0 to 30% fat; and 1 to 85% water. The product can be fluid or have the viscosity and texture of a pap. In one embodiment the product is in a dry or substantially dry powder form.

In a fourth aspect, the invention provides a method for inducing specific oral tolerance to cereal protein in a young mammal with allergy to said cereal protein comprising feeding to the young mammal a therapeutic amount of a partial hydrolysate of cereal protein having a degree of hydrolysis between 9 and 18%

According to the first, second, third and fourth aspects of the invention, the degree of hydrolysis of the partial hydrolysate of cereal protein is preferably between 10 and 16%, and even more preferable between 11 and 13%.

According to the first, second, third and fourth aspects of the invention, preferably, the allergy is an IgE-mediated allergy.

According to the first, second, third and fourth aspects of the invention, preferably, the cereal is wheat, rice, or a mixture of wheat and rice.

### Brief Description of the Drawings

Figure 1 shows an SDS-PAGE analysis of intact and hydrolysed rice proteins;
Figure 2 shows an SDS-PAGE analysis of intact and hydrolysed wheat proteins;
Figure 3 shows the residual allergenicity of hydrolysed rice proteins according to the invention;
Figure 4 shows the residual allergenicity of hydrolysed wheat proteins according to the invention;

### Detailed Description of the Invention

In this specification, the following terms have the following meanings:
"cereal protein" means any and all proteins of dietary value found in cereals such as rice, wheat, oats, corn, barley, rye and mixtures thereof;
"degree of hydrolysis" or "DH" of a protein means the number of peptide bonds in the intact protein which are cleaved during the hydrolysis divided by the number of peptide bonds in the intact protein expressed as a percentage;
"hypoallergenic (HA) cereal protein" means partially hydrolysed cereal protein the allergenicity of which is at least 100 times less than that of the intact cereal protein (by analogy with the provisions of EU Directive 96/4/EC relating to milk proteins);
"oral tolerance " means an active state of immunological hypo-responsiveness to antigens delivered via the oral route;
"specific oral tolerance induction" means partial or complete oral desensitisation of a young mammal having an allergy, preferable an IgE-mediated allergy to a cereal protein, or re-establishment of tolerance to said allergen
"with IgE-mediated allergy" means that an IgE-mediated allergy has been hypothesised or diagnosed, based on one or more of the following tests:
   - positive skin prick test (SPT) to an allergen, preferably cereal protein,
   - positive specific IgE to an allergen, preferably cereal protein,
   - immediate positive reaction (IgE) to an allergen, preferably cereal protein, as shown by provocation (oral challenge) test
"primary prevention of allergic reactions to cereal protein" means prevention of establishment of such an allergic reaction and includes reduction of risk of such an allergic reaction;
"purified protease" means a protease which is not contaminated by other enzymes capable of hydrolysing carbohydrates such as alpha-amylase;
"sugar" means a carbohydrate used in cereal products to provide a sweet taste including but not limited to sucrose, glucose and fructose;

All references to percentages are percentages by weight unless otherwise stated. "Percentage of dry weight" in the context of a specific ingredient in a product according to the invention means the amount of that ingredient expressed as a percentage of total dry matter in the product.

The invention uses a partial hydrolysate of cereal protein having a DH between 9 and 18%, preferably between 10 and 16%. The cereal may be any cereal used for dietary purposes including rice, wheat, rye, corn, barley, oats or a mixture thereof. Preferably, the cereal is wheat, rice, or a mixture of wheat and rice.

The partial hydrolysate of cereal protein may have an allergenicity which is reduced by a factor of at least 100 compared to the intact cereal protein as measured by the technique described by Fritsché et al (Int. Arch. Aller and Appl Imm., 93, 289-293, 1990). These partially hydrolysed cereal proteins and products containing them may thus be described as hypoallergenic.

The partial hydrolysate of cereal protein may be produced by any suitable method known in the art. A suitable starting material is cereal flour, for example wheat flour or rice flour. The granulometry of the flour is not critical and the particle size may vary between 200 and 500 microns for example.

An example of a process for making a partial cereal hydrolysate for use according to the invention is as follows:- the cereal flour is mixed with water and the mixture is heated to a temperature in the range from 60 to 65°C for 10 minutes, then cooled to 55°C. A protease such as the bacterial serine endoprotease subtilisin (sold, for example under the trade mark Alcalase®) is added and the mixture is maintained at 55°C for two hours. Then the temperature is raised to 70 to 75°C and held there for 10 minutes. The mixture is cooled to 55°C again and a different protease such as a mixture of bacterial proteases from *Bacillus amyloliquefaciens* and *Bacillus licheniformis* (available from Novozymes A/S Bagsvaerd, Denmark under the trade mark Protamex®) is added. The mixture is maintained at 55°C for a further two hours then the temperature is raised to between 85 and 95°C and held there for a period of 30 minutes to inactivate the enzymes and terminate the hydrolysis. The partially hydrolysed cereal proteins thus obtained are in the form of a liquid and may be used in this state or may be dried by any suitable technique known in the art such as roller drying, spray drying or extrusion.

The partial cereal hydrolysate or products made there from can be further processed by an extrusion step. Any generally know in the art extrusion process and equipment can be used. However preferably the extrusion step is conducteded or performed with a twin screw extruder, at a speed of 200 to 260rpm, at a temperature between 130 °C and 150°C, with a product flow rate of between 6 and 8 kg/hour, a water flow rate of 7-11ml/min and a pressure of between 50 to 150 bars. The extrusion step allows for obtaining a particular texture of the product, having particular physical characteristics such as dissolution time, viscosity once reconstituted and the like.

A particularly preferred process for producing a partial hydrolysate of cereal proteins for use according to the invention comprises mixing cereal flour with water and buffering agent (preferably at a pH of between 7.0 and 8.0), carrying out a preliminary (first) heat treatment (however optional), adding a purified protease and maintaining the mixture at a temperature between 40 and 80°C or 40°C to 70°C (second heat treatment), for from 30 minutes to 240 minutes to obtain a partial hydrolysate having a degree of hydrolysis between 9 and 18%.

In one embodiment, the preliminary (first) heat treatment is performed at between 40°C to 90°C, preferably between 60°C and 80 °C for a period of time of between 1 and 8 hours, preferably between 2 and 3 hours.

In one embodiment, the second heat treatment above is replaced by a progressive heat treatment: The temperature is slowly raised from ambient to 70°C or 80°C over a period of time of between 2 and 5 hours, preferably between 3 and 4 hours. The rate of temperature increase can be between 0.05°C/min and 0.40°C/min, preferably between 0.08 and 0.3 °C/min and most preferably between 0.10°C/min and 0.15°C/min. Optionally, a plateau phase can be used to maintain the hydrolysate at the final temperature of the heat treatment during a further period of time (1, 2,3 or 5 hours).

The optimal temperature for the heat treatment(s) is highly dependent of the nature of the cereal proteins and of the enzyme used. Above 70°C it has been noticed that the enzymatic activity may decrease.

The purified protease may be a bacterial protease which is not contaminated with alpha-amylase such as the enzyme sold under the trade mark Alcalase AF® (Novozymes A/S Bagsvaerd, Denmark) or a protease of animal origin such as trypsin.

As will be appreciated by those skilled in the art, the purpose of the preliminary heat treatment is to promote unfolding of the protein molecules rendering them more accessible for the subsequent enzymatic hydrolysis. The preliminary heat treatment may comprise for example heating at a temperature between 50 and 130°C for a time between 15 seconds to 10 minutes in a heat exchanger or with direct injection of steam.

Preferably the hydrolysis is conducted at a temperature between 50 and 80°C and for a period between 30 and 180 minutes, preferably between 60 mins and 120 mins. Longer period of time, such as 4 or 5 hours may however also be considered. In some instances very short period of time (10 or 15 mins) may be beneficial.

The partial hydrolysates of cereal protein according to the invention may be used in place of intact cereal protein in food products for young mammals such as human infants and toddlers as well as the young of companion animals such as dogs and cats.

The invention uses products comprising, by percentage weight of dry matter, 1 to 100% cereal flour, preferably 15% to 100%, in which the protein is partially hydrolysed and has a degree of hydrolysis between 9 and 18%, 0 to 40% sugar(s), and 0 to 30% fat; and 1 to 85% water.

The products can comprise starch, native or hydrolyzed. Hydrolyzed starch may accrue the amount of hydrolyzed protein cereal.

The products can be in a fluid (liquid) form. These can be sold ready to consume (without further dilution).

The products can be in the form of dehydrated powders which are prepared for consumption by reconstitution with water or milk.

The products can be in the form of extruded snack products, especially intended for consumption by toddlers.

When the product is in the form of a dehydrated instant powder reconstituted with liquid (water or milk) or of a liquid cereal products, the viscosity range covered by the invention is from 0 mPa·s to 4000 mPa·s, preferably between 1000 and 3500mPa·s. Preferably, for weaning food, the viscosity reached is between 2000 to 3000 mPa·s. The viscosity measurement can be performed by any known standard method. The values indicated above were measured at 60°C, 50 rpm, 10mins on a product diluted with water at 15.6% solid.

A dehydrated cereal powder for use according to the invention preferably contains 70 to 80% of the cereal flour, 10 to 30% sugar (preferably 15% to 20%), 2 to 10% fat and 1 to 3% water (preferably 2% to 3%). Such products may also include up to 20% starch. They are sold in dehydrated form and are rehydrated by mixing with liquid milk (or a mixture of powdered milk and water) to form a pap ready for consumption.

Preferably, the pap is prepared using a mixture of the cereal product as described above, for use according to the invention, water and powdered hypoallergenic infant formula i.e. infant formula containing partially hydrolysed milk proteins and complying with the requirements of Directive 96/4/EC. Alternatively, ready to feed liquid HA infant formula may be used to reconstitute the cereal product if available. This has the advantage of providing a cereal-based weaning food with substantially reduced allergenicity as all proteins in the food have been subjected to partial hydrolysis.

The pap can also be prepared by mixing the cereal product according to the invention and other hypoallergenic preparations containing proteins.

In one embodiment, the partial hydrolysate of or partially hydrolysed cereal proteins of the invention is provided together with an hypoallergenic egg preparation.

In another embodiment, the other hypoallergenic preparation can comprise hypoallergenic milk or hypoallergenic infant formula.

In yet another embodiment the partial hydrolysate or partially hydrolysed of cereal proteins of the invention comprises both an hypoallergenic egg preparation and an hypoallergenic milk or infant formula.

The hypoallergenic egg preparation can be as described in patent publications EP1867237A1 and WO2007/144397A1.

The hypoallergenic infant formula can be infant formula containing partially hydrolyzed milk proteins and complying with the requirements of Directive 96/4/EC. This has the advantage of providing a cereal-egg based weaning food with substantially reduced allergenicity as all proteins in the food have been subjected to partial hydrolysis.

In another embodiment, the cereal product further contains milk solids. Such a product may be prepared for consumption simply by mixing with water. Again, the milk solids if present are preferably provided by hypoallergenic infant formula.

A liquid cereal product for use according to the invention preferably comprises 5 to 10% of the cereal flour , 2 to 7% milk solids, up to 15% fat, up to 30% sugar, up to 5% starch and 75 to 90% water. Such products may be designated cereal milk drinks and are typically sold ready to consume in single serve cartons or bottles. The milk solids are preferably provided by hypoallergenic infant formula.

Cereal products may contain starch. If starch is present, it may be either native starch or partially hydrolysed starch prepared as known in the art, for example from US Patent No. 4,374,860. The degree of starch hydrolysis could be comprised between DE 5 to 55 or between DE 20 to 45 and finally between DE 25 to 35. The degree of starch hydrolysis was optimized to obtain the right sensory and physicochemical properties of the final application of the invention. If a product with partially hydrolysed starch as well as partially hydrolysed proteins is desired, the cereal flour used as the starting material may be subject to two hydrolyses, one directed to the starch and the other to the protein. These hydrolyses may be carried out simultaneously or sequentially.

In addition to cereal products such as those described above, the partial hydrolysate of cereal protein may also be used as an ingredient, for example in the form of cereal flour in which the protein is partially hydrolysed and has a degree of hydrolysis between 9 and 18%, in any food product that conventionally contains cereal flour such as pasta, bread, cakes biscuits etc.

As discussed above, the desensitisation to cereal protein relies upon the successful induction of oral tolerance to the protein. The present inventors believe that this in turn may be achieved by striking a balance between the residual antigenicity of the partially hydrolysed proteins and their capacity to induce oral tolerance. In general, the residual antigenicity of the hydrolysed protein should be at least 100 times less than that of the intact protein.

Proteins vary not only by molecular weight but also and in particular, by their sequence, degree of hydrophobicity - hydrophilicity, tri-dimensional structures, pKa and many other characteristics. As such the prediction of the oral tolerance induction is difficult, if not impossible. Furthermore the profile/potential of allergenicity greatly varies between and among families of proteins. Also the protein / host interactions, and reaction of the immune system can be very different between proteins. As such, the knowledge on milk proteins for example, can not directly apply to cereal proteins.

The invention will now be further described with reference to the following examples.

### Preparation of partial Cereal Hydrolysates

### Example 1

10Kg of rice flour (pre-treated according to the US Patent 4,374,860 to partially hydrolyse the carbohydrate content) and 23kg of water were mixed and heated at 55°C. A solution of buffering reagent (Na(OH)₂ or K(OH) ₂ or Ca(OH)₂) was prepared and added to the mixture for pH adjustment. 5% of Protamex® enzymes (batch PW2A1006, Novozymes A/S Bagsvaerd, Denmark) was added and the mixture was maintained at 55°C for 2 hours. After this first hydrolysis step the mixture was heated at 90°C for 10 min. The mixture was then cooled to 55°C, 5% of Flavourzyme® 1000 L enzymes (batch 400904, Novozymes A/S Bagsvaerd, Denmark) was added and the mixture was maintained at 55°C for 2 hours. After this second hydrolysis step, the mixture was heated at 90°C for 30 min and then spray-dried to obtain a powder containing partially hydrolysed rice proteins with a DH of 14.2% which was conditioned in an aluminium bag.

### Example 2

10Kg of rice flour (pre-treated according to the US Patent 4,374,860 to partially hydrolyse the carbohydrate content) and 23kg of water were mixed and heated at 55°C. A solution of buffering reagent (Na(OH)₂ or K(OH) ₂ or Ca(OH)₂) was prepared and added to the mixture for pH adjustment (to a pH of 7.8). 10% of Alcalase®2.4L enzymes (batch 500357, Novozymes A/S Bagsvaerd, Denmark) was added and the mixture was maintained at 55°C for 2 hours. After this first hydrolysis step the mixture was heated at 90°C for 10 min. The mixture was then cooled to 55°C, a further 10% of Alcalase enzymes was added and the mixture was maintained at 55°C for 2 hours. After this second hydrolysis step, the mixture was heated at 90°C for 30 min and then spray-dried to obtain a powder containing partially hydrolysed rice proteins with a DH of 15.9% which was conditioned in an aluminium bag.

### Example 3

10Kg of rice flour (pre-treated according to the US Patent 4,374,860 to partially hydrolyse the carbohydrate content) and 23kg of water were mixed and heated at 55°C. A solution of buffering reagent (Na(OH)₂ or K(OH) ₂ or Ca(OH)₂) was prepared and added to the mixture for pH adjustment (pH 7.8). 5% of Alcalase®2.4L enzymes (batch 500357, Novozymes A/S Bagsvaerd, Denmark) was added and the mixture was maintained at 55°C for 2 hours. After this first hydrolysis step the mixture was heated at 90°C for 10 min. The mixture was then cooled to 55°C, a further 5% of Protamex® enzymes (batch PW2A1006, Novozymes A/S Bagsvaerd, Denmark) was added and the mixture was maintained at 55°C for 2 hours. After this second hydrolysis step, the mixture was heated at 90°C for 30 min and then spray-dried to obtain a powder containing partially hydrolysed rice proteins with a DH of 11.2% which was conditioned in an aluminium bag.

### Example 4

15g of wheat flour (pre-treated according to the US Patent 4,374,860 to partially hydrolyse the carbohydrate content) and 70kg of water were mixed and heated at 55°C. A solution of buffering reagent (Na(OH)₂ or K(OH)₂ or Ca(OH)₂) was prepared and added to the mixture for pH adjustment. 5% of Protamex® enzymes (batch PW2A1006, Novozymes A/S Bagsvaerd, Denmark) was added and the mixture was maintained at 55°C for 2 hours. After this first hydrolysis step the mixture was heated at 90°C for 10 min. The mixture was then cooled to 55°C, 5% of Flavourzyme® 1000 L enzymes (batch 400904, Novozymes A/S Bagsvaerd, Denmark) was added and the mixture was maintained at 55°C for 2 hours. After this second hydrolysis step, the mixture was heated at 90°C for 30 min and then spray-dried to obtain a powder containing partially hydrolysed wheat proteins with a DH of 12.7% which was conditioned in an aluminium bag.

### Example 5 (not part of the invention)

15Kg of wheat flour (pre-treated according to the US Patent 4,374,860 to partially hydrolyse the carbohydrate content) and 70kg of water were mixed and heated at 55°C. A solution of buffering reagent (Na(OH)₂ or K(OH) ₂ or Ca(OH)₂) was prepared and added to the mixture for pH adjustment. 10% of Alcalase®2.4L enzymes (batch 500357, Novozymes A/S Bagsvaerd, Denmark) was added and the mixture was maintained at 55°C for 2 hours. After this first hydrolysis step the mixture was heated at 90°C for 10 min. The mixture was then cooled to 55°C, a further 10% of Alcalase enzymes was added and the mixture was maintained at 55 °C for 2 hours. After this second hydrolysis step, the mixture was heated at 90°C for 30 min, subjected to ultrafiltration using a 4kDa membrane and then spray-dried to obtain a powder containing extensively hydrolysed wheat proteins with a DH of 20.0% which was conditioned in an aluminium bag.

### Example 6

15Kg of rice flour, 70kg of water and a solution of buffering reagent (Na(OH)₂ or K(OH) ₂ or Ca(OH)₂) for pH adjustment were mixed and heated by steam injection for few seconds. 5% of Alcalase®2.4L AF enzymes (batch RBN00013, Novozymes A/S Bagsvaerd, Denmark) was added and the mixture was maintained at 60°C for 1 hour. After this hydrolysis step the mixture was again heated by steam injection for few seconds. The mixture was then roller-dried to obtain a powder containing partially hydrolysed rice proteins with a DH of 13.2% which was conditioned in an aluminium bag.

### Example 7

15Kg of wheat flour, 70kg of water and a solution of buffering reagent (Na(OH)₂ or K(OH) ₂ or Ca(OH)₂) for pH adjustment were mixed and heated by steam injection for few seconds. 5% of Alcalase®2.4L AF enzymes (batch RBN00013, Novozymes A/S Bagsvaerd, Denmark) was added and the mixture was maintained at 60°C for 2 hours. After this hydrolysis step the mixture was again heated by steam injection for few seconds. The mixture was then roller-dried to obtain a powder containing partially hydrolysed wheat proteins with a DH of 11% which was conditioned in an aluminium bag.

### Example 8

15Kg of a cereal flour mix (rice, wheat), 70kg of water and a solution of buffering reagent (Na(OH)₂ or K(OH) ₂ or Ca(OH)₂) for pH adjustment were mixed and heated by steam injection for few seconds. 5% of Alcalase®2.4L AF enzymes (batch RBN00013, Novozymes A/S Bagksvaerd, Denmark) was added and the mixture was pumped through continuous pipes at 60°C for a holding time of 1 hour. After this hydrolysis step the mixture was again heated by steam injection for few seconds. The mixture was then spray-dried to obtain a powder containing partially hydrolysed rice and cereal proteins with a DH of 9.2% which was conditioned in an aluminium bag.

### Example 9

25Kg of wheat flour and 70kg of water were mixed and heated at 130°C. 5% of Alcalase®2.4L AF enzymes (batch RBN00013batch 500357, Novozymes A/S Bagsvaerd, Denmark) was added and the mixture temperature was increased from 60 to 80°C for 3 hours (~0,11°C/min). After this hydrolysis step the mixture was heated at 90°C for 15 min. The mixture was then cooled to 60°C for formulation step, then roller-dried to obtain a powder containing partially hydrolysed wheat proteins which was conditioned in an aluminium bag.

### Example 10

15Kg of cereal flour (rice, wheat) and milk protein and 70kg of water and a solution of buffering reagent (Na(OH)₂ or K(OH) ₂ or Ca(OH)₂) for pH adjustment were mixed and heated by steam injection for few seconds, 5% of Alcalase®2.4L AF enzymes (batch RBN00013, NOVOZYMES A/S Bagsvaerd, Denmark) was added and the mixture was maintained at 60°C for 1 hour. After this hydrolysis step the mixture was heated by steam injection for few seconds. The mixture was then roller-dried to obtain a hydrolysed cereal powder which was conditioned in an aluminium bag.

### Example 11

20Kg of wheat flour (pre-treated according to the US Patent 4,374,860 to partially hydrolyse the carbohydrate content) and 30kg of water and a solution of buffering reagent (Na(OH)₂ or K(OH) ₂ or Ca(OH)₂) for pH adjustment, 5 % Alcalase®2.4L AF enzymes (batch RBN00013, NOVOZYMES A/S Bagsvaerd, Denmark) was added and the mixture was maintained at 60°C for 2 hours. After this hydrolysis step the mixture was heated to 90°C for 15minutes. Sucrose, starch and fat were added to the mixture; the final mixture was heat treated by steam injection for few seconds and then roller-dried and milled to obtain a hydrolysed cereal powder which was conditioned in an aluminium bag.

### Example 12

20Kg of wheat flour (pre-treated according to the US Patent 4,374,860 to partially hydrolyse the carbohydrate content) and 30kg of water and a solution of buffering reagent (Na(OH)₂ or K(OH) ₂ or Ca(OH)₂) for pH adjustment were mixed with 4kg of untreated wheat flour and heated by steam injection for few seconds; 5 % Alcalase®2.4L AF enzymes (batch RBN00013, NOVOZYMES A/S Bagsvaerd, Denmark) was added and the mixture was maintained at 60°C for 2 hours. After this hydrolysis step the mixture was heated to 90°C for 15minutes. Sucrose, starch and fat were added to the mixture; the final mixture was heat treated by steam injection for few seconds and then roller-dried and milled to obtain a hydrolysed cereal powder which was conditioned in an aluminium bag.

### Cereal Products containing Partial Hydrolysates of Cereal Proteins

The cereal products mentioned in the description and examples may be used in a method for inducing specific oral tolerance to cereal protein in a young mammal with allergy to said cereal protein. Such a method comprises feeding to the young mammal a therapeutic amount of a partial hydrolysate of cereal protein having a degree of hydrolysis between 9 and 18%. Preferably, the young mammal is a human infant, aged between 1 and 12 years old.

Usually, specific oral tolerance induction comprises the following steps:
- Identify the "maintenance dose" of the young mammal, i.e. the maximum dose of partial hydrolysate of cereal protein tolerated without provoking an allergic reaction. In order to identify the maintenance dose, increasing amounts of partial hydrolysate of cereal protein are fed to the young mammal, starting at very low dosage (for instance 0.2 g of cereal product), up to the an amount equivalent to the usual daily oral intake. This steps should be performed under medical supervision.
- Feed the young mammal daily with the maintenance dose for a period of time of one to a few months, for instance 2 to 10 months, usually for about 4 to 6 months. Preferably, the maintenance dose should be given in one serve per day. The cereal product can be fed dissolved in a water, or milk. It can also be mixed with food such as apple puree, yogurt, fruit juice, or soup.

The cereal product may contain from 7 to 10 g protein / 100 g cereal product, preferably from 8 to 9.5 g protein / 100 g cereal product.

In order to check if specific oral tolerance induction has been achieved, it is possible to challenge the young mammal with native cereal protein, at low dosage, under medical supervision.

### Example 13

An example of the composition of a cereal product is as follows (weight of ingredients other than water are given on a dry matter basis):-

| | |
|---|---|
| Rice flour with protein content partially hydrolysed (Example 6) | 60% |
| Sugar | 12% |
| Native potato starch | 15% |
| Fat mix | 10% |
| Vitamin/mineral premix | 0.5% |
| Water | 2.5% |

### Example 14

Another example of the composition of a cereal product is as follows (weight of ingredients other than water are given on a dry matter basis):-

| | |
|---|---|
| Rice flour with protein content partially hydrolysed (Example 6) | 75% |
| Sugar | 15% |
| Fat mix | 7% |
| Vitamin/mineral premix | 0.5% |
| Water | 2.5% |

The products of Examples 13 and 14 may be made by mixing the product obtained in Example 6 (for Example 13) or Examples 3 and 4 (for Example 14) omitting the final drying step in each case with the fat and sugar. The mixture is heat treated, roller dried and milled as for a conventional cereal product. Any heat or moisture sensitive ingredients such as vitamins or probiotics could be added at this point by dry mixing. Non replicating probiotics can also be added.

### Example 15

Another example of the composition of a cereal product is as follows (weight of ingredients other than water are given on a dry matter basis):-

| | |
|---|---|
| Wheat flour with protein content partially hydrolysed (Example 7) | 75% |
| Sugar | 15% |
| Fat mix | 7% |
| Vitamin/mineral premix | 0.5% |
| Water | 2.5% |

### Example 16

An example of the composition of a liquid cereal product is as follows (weight of ingredients other than water specified as percentage of dry matter):-

| | |
|---|---|
| Rice flour with protein content partially hydrolysed (Example 3) | 0.5% |
| Wheat flour with protein content partially hydrolysed (Example 4) | 5% |
| HA infant formula | 12.5% |
| Native starch | 1.5% |
| Vitamin/mineral premix | 0.5% |
| Water | 80% |

### Example 17

An example of the composition of a liquid cereal product is as follows (weight of ingredients other than water specified as percentage of dry matter):-

| | |
|---|---|
| Rice flour with protein content partially hydrolysed (Example 3) | 0.5% |
| Wheat flour with protein content partially hydrolysed (Example 4) | 5% |
| Hydrolysed whey protein | 1.5% |
| Fat mix | 3.5% |
| Lactose | 7.5% |
| Native starch | 1.5% |
| Vitamin/mineral premix | 0.5% |
| Water | 80% |

The products of Examples 16 and 17 may be made by mixing in water the partially hydrolysed rice and wheat flours with the hydrolysed whey proteins, the lactose, the starch and the mineral and vitamin premixes. Then, the product mix is pre-warmed to 70°C, the fat mix is added in line (Example 17 only) and the product is UHT treated, then cooled. During the cooling phase the product is homogenised at 250/50 bar then further cooled to ambient temperature before being packed into cartons or the like containers under aseptic conditions.

### Example 18

Another example of the composition of a cereal product is as follows (weight of ingredients other than water are given on a dry matter basis):-

| | |
|---|---|
| Wheat flour with protein and starch content partially hydrolysed (Ex. 12) | 75% |
| Potato starch | 19% |
| Fat mix | 2% |
| Enzymes | 0.5% |
| Vitamin/mineral premix | 0.4% |
| Buffering salt | 0.1% |
| Water | 3% |

### Example 19

Another example of the composition of a cereal product is as follows (weight of ingredients other than water are given on a dry matter basis):-

| | |
|---|---|
| Wheat flour with protein partially hydrolysed (Example 4) | 50 % |
| Milk with protein partially hydrolysed (as per Patent US5039532) | 20% |
| Egg with protein partially hydolysed (as per Patent WO2007/144397A1) | 10% |
| Potato starch | 14% |
| Fat mix | 2% |
| Enzymes | 0.5% |
| Vitamin/mineral premix | 0.4% |
| Buffering salt | 0.1% |
| Water | 3% |

### Example 20

Another example of the composition of a cereal product is as follows (weight of ingredients other than water are given on a dry matter basis):-

| | |
|---|---|
| Fat mix | 2% |
| Enzymes | 0.5% |
| Vitamin/mineral premix | 0.4% |
| Buffering salt | 0.1% |
| Water | 3% |

The compositions of examples 13 to 20 may be used for inducing specific oral tolerance to wheat, or rice, in young mammals with allergy to wheat or rice proteins.

### Analysis of proteins / peptides by gel electrophoresis

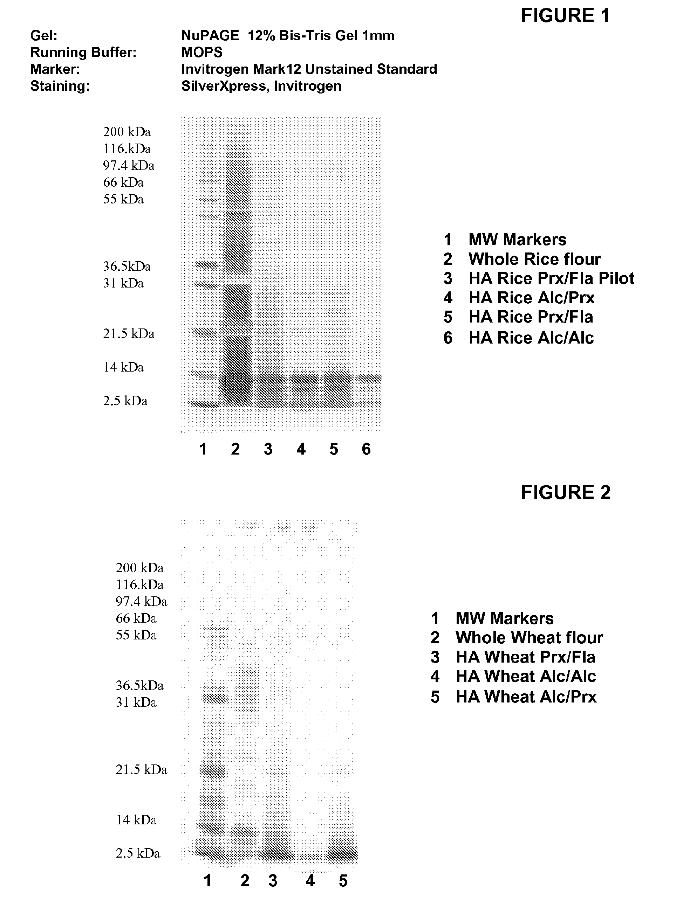

The effect of enzymatic hydrolysis on proteins in rice and wheat flour was examined by SDS-polyacrylamide gel electrophoresis (SDS-PAGE) using NuPAGE 12% Bis-Tris gels and Silverstaining. The results are shown in Figures 1 (rice) and 2 (wheat). In Figure 1, lane 5 corresponds to the hydrolysate of Example 1, lane 6 to the hydrolysate of Example 2 and lane 4 to the hydrolysate of Example 3. In Figure 2, lane 3 corresponds to the hydrolysate of Example 4 and lane 4 to the hydrolysate of Example 5. These figures show that in all cases the hydrolysis process largely eliminates protein bands in the partially hydrolysed products as compared to the intact rice or wheat flours.

Total nitrogen in the hydrolysates was determined by the Kjeldahl procedure. Degree of hydrolysis was measured by the TNBS method according to Adler-Nissen (J. Agric. Food. Chem. 27: 1256-1262, 1979).

### Residual Allergenicity of Cereal Hydrolysates

A functional *in vitro* assay of tritiated serotonin release from sensitised rat mast cells was used to determine IgE dependent allergenicity of potentially antigenic molecules from rice and wheat before and after partial hydrolysis. In the case of rice, the whole protein fraction was used, in the case of wheat, the investigation focused on gliadin, a wheat protein known to be implicated in the development of wheat allergy.

Briefly, the method is the following: culture RBL-2H3 cells in RPMI in 250 ml flasks. For the degranulation assay, remove culture medium, add 4 ml trypsin-EDTA to the cell layer and incubate 10 min. at 37°C. Shake slightly to detach cells, add 6 ml RPMI and mix. Dispense 9.5 ml in a 15 ml blue-top Falcon tube; centrifuge 5 min. at 310 g (Sorvall, rotor 75006445). Discard supernatant, add 10 ml RPMI to cell pellet, mix and centrifuge 5 min. at 310 g. Discard supernatant, add 10 ml RPMI to cell pellet, mix, count cells and re-suspend in RPMI at 4x105/ml. Dispense 100µl of this cell suspension into wells of a 96 wells Costar plate. Incubate the plate overnight at 37°C.

Remove 50 µl of culture medium from each well and replace with 50 µl of a rat anti-allergen (gliadin/rice protein) IgE antiserum containing 1 µl 3H Serotonin; incubate 2h at 37°C. Wash gently all wells twice with 250 µl HBSS, remove completely the supernatant and add 150 µl of allergen standard /sample /HBSS/ TritonX diluted in HBSS. Incubate 45 min. at 37°C, centrifuge 8 min. at 140 g (Mistral 2000) and transfer 50 µl of supernatant into an Optiplate. Add 200 µl of Microsint 40, mix and count radioactivity (Topcount Packard β-counter).

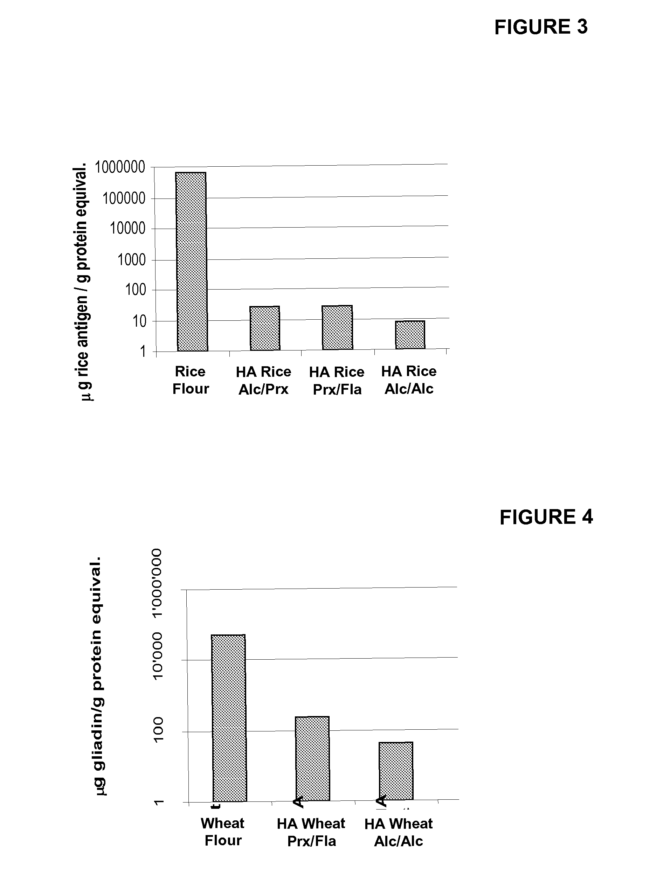

The results are shown in Figures 3 and 4, from which it may be seen that the partially hydrolysed cereals had a much reduced allergenicity compared to the corresponding intact cereal protein:
8-25 µg rice protein antigen /g protein.
25-175 µg gliadin/g protein.

Results are expressed as amount of rice protein / gliadin able to trigger mast cells per gram of protein.

In Figure 3, moving from left to right, the second bar was obtained from the hydrolysate of Example 3, the third bar was obtained from the hydrolysate of Example 1 and the fourth bar was obtained from the hydrolysate of Example 2. In Figure 4 moving from left to right, the second bar was obtained from the hydrolysate of Example 4 and the third bar was obtained from the hydrolysate of Example 5.

## Claims

1. A partial hydrolysate of cereal protein for use in inducing specific oral tolerance in a young mammal with allergy to said cereal protein, wherein the hydrolysate has a degree of hydrolysis between 9 and 18%.

2. A hydrolysate for use according to Claim 1, wherein the degree of hydrolysis is between 10 and 16%.

3. A hydrolysate for use according to Claim 1 or 2, wherein the cereal is rice, wheat, rye, corn, barley, oats or a mixture thereof.

4. A cereal flour for use in inducing specific oral tolerance in a young mammal with allergy to said cereal protein, in which the protein is partially hydrolysed and has a degree of hydrolysis between 9 and 18%.

5. A cereal flour for use according to Claim 4, wherein the degree of hydrolysis is between 10 and 16%.

6. A cereal product for use in inducing specific oral tolerance in a young mammal with allergy to said cereal protein, said product comprising, by percentage weight of dry matter, 15 to 100% cereal flour according to Claim 4 or 5, 0 to 40% sugar, 0 to 30% starch and 0 to 30% fat; and 1 to 85% water.

7. A cereal product for use according to claim 6 having a viscosity between 2000 and 3000 mPa s at 60°C/50rpm.

8. A cereal product for use according to claim 6 or 7 wherein said cereal product comprises hypoallergenic egg proteins.

9. A cereal product for use according to any of claims 6 to 8 comprising by percentage weight of dry matter 5 to 10% cereal flour according to Claim 4 or 5, 2 to 7% milk solids, up to 15% fat, up to 30% sugar and up to 5% starch; and 75 to 90% water.

10. A cereal product for use according to Claim 9, wherein the milk solids are provided by hypoallergenic infant formula.

11. A cereal product for use according to any of Claims 5 to 10, wherein the cereal is rice, wheat, rye, oat, barley or a mixture thereof.

12. A method for inducing specific oral tolerance to cereal protein in a young mammal with allergy to said cereal protein comprising feeding to the young mammal a therapeutic amount of a partial hydrolysate of cereal protein having a degree of hydrolysis between 9 and 18%.

13. A method as claimed in Claim 12, wherein the young mammal is a human infant.

14. A method as claimed in any of Claims 12 or 13, wherein the degree of hydrolysis is between 10 and 16%.
